# EUROPEAN PATENT APPLICATION

(11) **EP 3 760 710 A1**
(43) Date of publication of application: **06.01.2021**
(21) Application number: 19761610.5
(22) Date of filing: 27.02.2019
(51) Int. Cl.: C12N 5/0775

(54) **METHOD FOR ISOLATING AND EXTRACTING ADIPOSE-DERIVED STEM CELLS FROM ADIPOSE TISSUE AND CULTURING SAME WITHOUT USING COLLAGENASE, AND KIT FOR ISOLATING AND EXTRACTING ADIPOSE-DERIVED STEM CELLS**

(30) Priority: 27.02.2018 US 201862636056 P
(71) Applicant: University of The Ryukyus, Nakagami-gun, Okinawa 903-0213 (JP); Orthorebirth Co., Ltd., Yokohama-shi, Kanagawa 224-0033 (JP)
(72) Inventor: SUNAMI, Hiroshi, Nakagami-gun, Okinawa 903-0215 (JP); SHIMIZU, Yusuke, Nakagami-gun, Okinawa 903-0215 (JP); FUTENMA, Naoko, Nakagami-gun, Okinawa 903-0215 (JP); MAKITA, Masashi, Yokohama-shi Kanagawa 224-0033 (JP); OSAKA,Naoya, Okinawa 9030213 (JP)
(74) Representative: Provvisionato, Paolo
(86) International application number: PCT/JP2019/007472
(87) International publication number: WO 2019/168000

(57) **Abstract**

Adipose-derived stem cells are isolated and extracted from adipose tissue without using collagenase. By placing a nonwoven fabric sheet having enough space between the fibers on adipose tissue mass and pressing the sheet against the adipose tissue mass with appropriate force, the adipose tissue is entered into the gap space between the fibers so that the adipose tissue is brought into close contact with surrounding fibers. By immersing the nonwoven fabric sheet in a culture medium in that state, a large number of adipose-derived stem cells crawl out from the adipose tissue and adhere on the surface of the fibers.

## Description

### Technical Field

The present invention relates to a method for isolating and extracting adipose-derived stem cells from adipose tissue without using a collagenase, and a kit for isolating and extracting adipose-derived stem cells used in the method.

### Background of Invention

A method of extracting adipose tissue-derived stem cells existing in adipose tissue from a patient and proliferating the adipose tissue-derived stem cells in large quantities at a CPC facility and injecting the proliferated adipose tissue-derived stem cells into a defect portion of the patient has been proposed (Patent Document 1).

As a method for extracting adipose derived stem cells from adipose tissue, collagen of the extracellular matrix is degraded by using collagenase so that the connections between adipose tissues is loosened. In adipose tissue, collagen acts as a glue that binds cells together. Therefore, by degrading the collagen, adipose tissue-derived stem cells that are embedded in adipose tissue can be extracted. While trypsin degrades protein, collagenase degrades only collagen. Therefore, relatively little damage is caused to cells during collagenase degradation.

However, because cells contain collagen as a component, collagenase can also damage the cells. Also, when collagen of cell is degraded using collagenase and adipose tissue-derived stem cells are isolated, a centrifuge needs be used to separate adipose-derived stem cells from lighter specific-gravity adipocytes, but centrifuge separation may cause physical damage to the cells. To minimize damage to cells that are for regenerative therapy, adipose tissue-derived stem cells need to be extracted from adipose tissue without using collagenase and centrifuge.

As a method of extracting adipose stem cells from adipose tissue without using collagenase, it has been proposed that the stem cells contained in adipose tissue is made crawl out from adipose tissue and adhered and cultured on a scaffold of nonwoven sheets. As a commercially available product that is used for this method, adipose stem cell separation kit of Bio Mirai Kobo Co., Ltd., is known (Non-Patent Document 1). According to the explanation of the company's adipose stem cell separation kit, it can extract and separate adipose-derived stem cells that produce abundant extracellular matrices by trapping and culturing adipose tissue on a three-dimensional structural substrate made of a nonwoven sheet having PE-PP core-sheath structure coated with HAp. The kit utilizes the nature of adipose tissues that they are difficult to adhere to flat-surface structures such as flasks, but are easily trapped in fibrous structures. By limiting the culture period, stem cells that proliferate faster than fibroblasts or vascular endothelial cells can be proliferated on the surface of fibers, and thus they can be separated in a high degree of purity.

### [Prior art documents]

### [Patent Documents]

[Patent Document 1] Japanese patent publication No. 2012-510279

### [Non-patent document]

[Non-Patent Document 1] Funakoshi Co., Ltd. Description of Adipose Stem Cell Separation Kit Products of Bio-Mirai Kobo

### Summary of Invention

### [Problem to be solved by the invention]

However, the above-mentioned adipose stem cell separation kit is used such that it just places seeded adipose tissue on a nonwoven fabric sheet. Thus, when it is immersed in a medium in that state, the adipose tissue may float on the medium. The number of stem cells that crawl out of from the adipose tissue toward the surface of the fibers is limited when the adipose tissue is simply loaded on a sheet without having tight contact.

### [Means to Solve the Problem]

Under such circumstances, the inventors of the present invention made an intensive study and found that stem cells contained in adipose tissue do not grow very much neither in vitro nor in vivo as long as they are existing in a soft adipose tissue. The reason may be that highly proliferative adipose stem cells are dormant in soft adipose tissues, maintaining tissue homeostasis. However, although the adipose-derived stem cells do not grow actively in a soft adipose tissue, if a hard scaffold is placed around the adipose tissue, the dormant adipose stem cells crawl out from the adipose tissue toward the scaffold and start proliferation explosively,

Taking this nature of cells into consideration, inventors of the present invention found that by pressing the scaffold material made of nonwoven sheets against adipose tissue, adhesion between the adipose tissue and scaffold material is increased so that mechanical stress is applied, then initial cell adhesion is dramatically improved, and as a result, adipose stem cells contained in the pressed adipose tissue proliferate in a large amount. Based on this finding, it has been discovered that when a nonwoven fabric sheet having a sufficient space between the fibers is placed on a adipose tissue mass and pressed with an appropriate force, the adipose tissue is entered between the fibers of the nonwoven fabric sheet and comes into contact with the fibers surrounding the adipose tissue. And when immersed in a medium in that state, a large number of stem cells are crawled out from the adipose tissue and adhere to the surface of the surrounding fibers.

Based on above findings, the inventors of the present invention reached following invention: A method of proliferating adipose derived stem cells contained in adipose tissue by extracting them without using a collagenase, the method comprises:
placing an adipose tissue mass containing adipose-derived stem cells that are collected from a body of a patient in a culture vessel, and covering the adipose tissue mass with a nonwoven fabric sheet formed of biodegradable fibers having an outer diameter of 10 µ m -100 µ m and a gap between the fibers is 10 µ m - 500 µ m, filling the culture vessel with culture medium for adipose derived stem cells so that the adipose tissue mass covered by the nonwoven sheet is immersed in the culture medium for adipose derived stem cells,
pressing the nonwoven fabric sheet against the adipose tissue mass so that adhesion between the adipose tissue mass and the nonwoven fabric sheet is increased, thereby promoting the adipose-derived stem cells contained in the adipocyte tissue mass to crawl out onto the surface of the biodegradable fibers, the adipose-derived stem cells crawled out from the adipose tissue is proliferated in a large amount to fill the gap space between fibers of the nonwoven fabric sheet,

Further, inventors of the present invention reached an invention of a kit for extracting adipose derived stem cells in adipose tissue for use in extracting and proliferating adipose derived stem cells in adipose tissue without using a collagenase, the kip comprises:
a container for cell culture, a cell strainer, a nonwoven fabric sheet, a plate dish or ring that is used to as a weight to press a nonwoven fabric sheet,
side and bottom surfaces of the cell strainer are meshed so that the cell culture medium can penetrate the cell strainer through the pores of the mesh,
the nonwoven fabric sheet is made of biodegradable fibers having an outer diameter of 10 µm to 100 µm, and a gap between the fibers and the fibers is 10 µm to 500 µm
an adipose tissue mass containing adipose derived stem cells is placed in a cell strainer, and a nonwoven fabric sheet is placed on the adipose tissue mass in the cell strainer, and then the culture vessel containing the cell strainer is filled with a culture medium for adipose derived stem cell culture so that the adipose tissue mass is immersed in the adipose derived stem cell culture medium in the culture vessel. by pressing the nonwoven fabric sheet covering the adipose tissue mass against the adipose tissue mass using the eye dish or ring so that the adhesion between the adipose tissue mass and the nonwoven fabric sheet is increased, the adipose-derived stem cells contained in the adipose cell tissue mass is promoted to crawl out to the surface of the biodegradable fibers, then the adipose-derived stem cells crawled out from the mass is proliferated in the space between the fibers of the nonwoven fabric sheet,

Preferably, the nonwoven fabric sheet has a thickness of from about 0.1 to 1.0mm. If the thickness of the sheet is less than 0.1mm, the stem cells are more likely to pass through the sheet and are less likely to be trapped in the sheet. If the nonwoven fabric sheet is too thick, it may be difficult to bend, fold, or handle the wound, and if the adipose derived stem cells are implanted in the body with the scaffold of the non-woven fabric sheet, the thicker, the more time it takes, and thus less desirable. In the event of producing a cell sheet by seeding adipose, if the nonwoven fabric sheet is too thick, it may take a long time for the stem cells to evenly spread across the nonwoven fabric sheet to form a uniform cell sheet.

Preferably, in the culture vessel, the adipose tissue mass is sandwiched between two nonwoven fabric sheets and immersed in the medium in a sandwich state to be cultured. By sandwiching the adipose tissue mass between two nonwoven fabric sheets, the stem cells can be grown between the fibers of the nonwoven fabric sheets and prevented from crawling out toward the culture container or the cell strainer.

Preferably, the biodegradable fibers constituting the nonwoven fabric sheet contain hydroxyapatite particles.

More preferably, outer diameter of the biodegradable fibers constituting the nonwoven fabric sheet is 30 µm to 60 µm.

Preferably, a cell non-adhesive dish plate may be used instead of the cell strainer, and the dish plate is filled with the medium for culturing adipose derived stem cells to conduct culturing. Cells bind specific proteins of the cell to the proteins adsorbed on a surface of substrate surface and adhere to the surface of the substrate. Therefore, if there is no protein on the surface of the substrate to which the cell can bind, the cell cannot adhere to the substrate. Dish plate coated with MPC polymers, superhydrophilic gels, etc. can be used as cell-non-adhesive dish plate because they become super-flooded surfaces and can reduce the adsorption of cell-adhesive proteins.

By using the method and kit of the present invention, a large amount of adipose derived stem cells can be proliferated between the fibers of a nonwoven fabric sheet made of biodegradable fibers.

By using the kit of the present invention, it becomes possible to implant adipose derived stem cells grown in large quantities between fibers of a nonwoven fabric sheet made of biodegradable fibers in a body of a patient together with the nonwoven fabric sheet scaffold.

By using the kit of the present invention, a weight such as an eye dish or a ring can be placed from above in a condition in which the nonwoven fabric sheet is covered from above or sandwiched. As a result, the adipose tissue does not float on the medium, and the adipose tissue and the nonwoven fabric sheet can be brought into close contact with each other by pressing with a weight.

By using the kit of the present invention, it becomes possible to grow a large amount of adipose derived stem cells in an initial culture of cell tissue. As a result, it becomes not necessary to perform subculture.

### BRIEF DESCRIPTION OF DRAWINGS

[Figure 1] FIG. 1 shows two kinds of methods for culturing adipose derived stem cells contained in adipose tissue masses using cell strainers.
[Figure 2] FIG. 2 shows culturing using a 6-well plate as a culture vessel.
[Figure 3] FIG. 3 shows culturing using a 10cm dish as a culture vessel.
[Figure 4] FIG 4 shows culturing using a 15cm dish as a culture vessel.
[Figure 5] FIG 5 shows a method of degassing a nonwoven fabric sheet that is used in an embodiment of the present invention.
[Figure 6] FIG 6 illustrates a flow of seeding adipose tissue onto a nonwoven fabric sheet using a kit of an embodiment of the present invention.
[Figure 7] FIG. 7 shows a tape affixed to a 6-well plate.
[Figure 8] FIG 8 shows a flow of adipose tissue seeding in a 6-well plate.

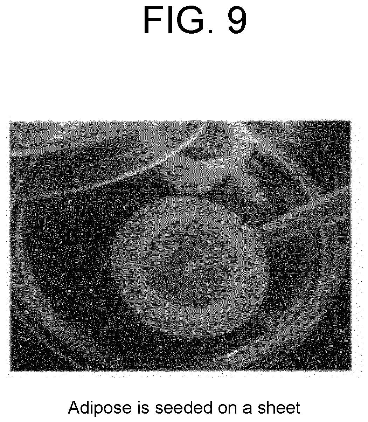

[Figure 9] FIG 9 shows a view of seeding adipose tissue using a pipette on a nonwoven fabric sheet that is used in an embodiment of the present invention.

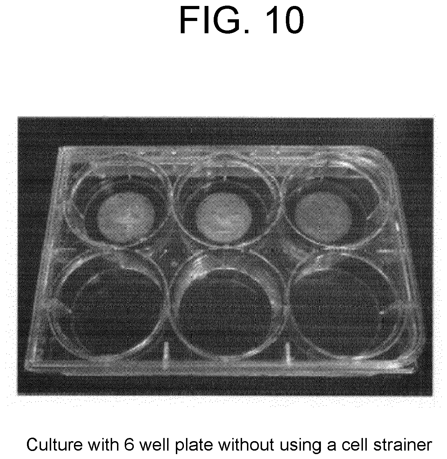

[Figure 10] FIG. 10 shows culturing using a 6-well plate without using a cell strainer.
[Figure 11] Fig.11 (A) is an electron micrograph showing fiber structure of the nonwoven fabric sheet before the culture according to an embodiment of the present invention. Fig.11 (B) is an electron micrograph showing the state that adipose-derived stem cells has grown densely spread to fill the space between the fibers of the nonwoven fabric sheet after the culture according to an embodiment of the present invention.
[Figure 12] FIG. 12 is an image showing the results of an experiment in which adipose stem cells were grown using the adipose stem cell separation kit of Bio Mirai KOBO Co., Ltd. FIG 12 (A) shows the condition in which adipocytes were seeded on the scaffold. FIG 12 (B) shows the condition in which adipocytes were grown on the scaffold.
[Figure 13] FIG 13 shows an embodiment of the present invention in which the nonwoven fabric sheet of an embodiment of the present invention is formed into a roll cake shape.
[Figure 14] FIG 14 shows a result of measuring the number of cultured cells adhered to a cell strainer, a nonwoven fabric sheet, and a eye dish respectively by absorbance (wavelength 440nm) in a method of culturing stem cells which is an embodiment of the present invention.
[Figure 15] FIG 15 is an SEM photograph showing the results of differentiation inducing of stem cells adhered to a nonwoven fabric sheet into chondrocytes using a method of culturing stem cells which is an embodiment of the present invention;
[Figure 16] FIG 16 is an SEM photograph showing the results of differentiation inducing of stem cells adhered to a nonwoven fabric sheet into vascular endothelial cells using a method of culturing stem cells which is an example of the present invention;
[Figure 17] FIG 17 is an SEM photograph showing the results of differentiation inducing of stem cells adhered to a nonwoven fabric sheet into adipocytes using a method of culturing stem cells which is an example of the present invention;
[Figure 18] FIG 18 is an SEM photograph showing the results of differentiation inducing of stem cells adhered to a nonwoven fabric sheet into adipocytes using a method of culturing stem cells which is an example of the present invention;

### Embodiment for Carrying Out the Invention

### <Nonwoven fabric sheet>

In the present invention, a nonwoven fabric sheet made of biodegradable fibers is used as a substrate serving as a scaffold for proliferating stem cells contained in adipose tissue. Since the fibers constituting the nonwoven fabric sheet of the present invention have an outer diameter of 10 to 100 µ m, sufficient space is formed between the fibers. And when the adipose tissue mass is seeded on the nonwoven fabric sheet of the present invention, the adipose tissue mass enters the space between the fibers, and in this state, the adipose tissue comes into contact with the surface of the fibers. In a preferred embodiment of the present invention, the distance between the fibers constituting the nonwoven fabric is from 10 µm to 500 µm.

The fibers constituting the nonwoven fabric sheet of the present invention can be preferably manufactured by depositing a plurality of fibers on a plane using an electrospinning method to form a sheet thereon. When an electrospinning method is used, a nonwoven fabric sheet can be formed by depositing the electrospun fibers on a rotary drum in a form of a sheet.

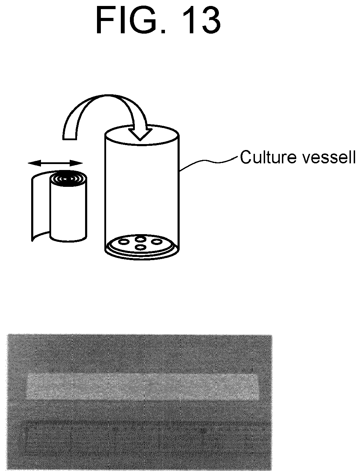

The nonwoven sheet formed in the form of a sheet is cut into a rectangle, and the ends of the rectangle are pinched and wound in the longitudinal direction, whereby the nonwoven sheet can be produced in the form of a roll cake as shown in FIG. 13. The roll cake shape enables compact cell culturing.

### <Biodegradable fiber>

As the fibers constituting the nonwoven fabric sheet of the present invention, a biodegradable resin such as polylactic acid or PLGA can be used. By using a biodegradable resin for producing the nonwoven fabric sheet of the present invention, it becomes possible to graft cells grown on the nonwoven fabric sheet scaffold in the human body by implanting the scaffold having proliferated cells in a human body.

In one embodiment of the present invention, the fibers constituting the nonwoven fabric sheet can be electrospun as a composite fiber of HAp and a biodegradable resin by spinning a spinning solution prepared by mixing HAp particles with a biodegradable resin dissolved in a solvent using an electrospinning method.

Considering that the fibers constituting the nonwoven fabric sheet have the seeded adipose tissue entered into the space between the fibers and trapped therein, the outer diameter of the fibers is preferably from about 10 to 100 µ m, more preferably from 30 µm to 60 µm.

### <Seeding of adipose tissue onto nonwoven sheet>

In one preferred embodiment of the present invention, adipose-derived stem cells are seeded onto a nonwoven fabric sheet by placing a nonwoven fabric sheet on a mass of adipose tissue collected from a body of a patient. Adipose tissue mass collected from a patient is soft and has an indefinite shape. When it is covered by a nonwoven fabric sheet placed thereon, it receives a pressure by the fibers of the nonwoven fabric sheet so that the adipose tissue mass flexibly changes its shape and enters into a gap between the fibers of the nonwoven fabric sheet. Stem cells are cultured by immersing the adipose tissue mass in a medium in a state in which the adipose tissue mass is sandwiched between fibers and trapped in a nonwoven fabric sheet.

Size of the adipose tissue mass is basically smaller than that of the sheet. It may be any size as long as it can be covered or sandwiched by sheets. Even if the size of the adipose tissue mass is larger than the sheet, it is not impossible to use the sheet. It is also possible to use the sheet such that a plurality of sheets are pasted onto a large adipose tissue. In an embodiment of the present invention, adipose tissue that is finely cut in a range of 1 - 5mm is used.

### <Hydroxyapatite>

In order to enhance cell adhesion to fibers of the nonwoven fabric sheet of the present invention, it is preferable to incorporate particles of hydroxyapatite (HAp) into the biodegradable fibers or to coat HAp on the surface of the fibers. Since HAp has good affinity with almost all of the adhesive cells, it can be suitably used for the fibers of the nonwoven fabric sheet of the present invention.

A nonwoven fabric sheet in which HAp is contained in a bioabsorbable fiber can be used as a scaffold material for cell proliferation. When the proliferated stem cells are implanted into the patient's body with a scaffold material, it is preferable that the outer diameter of HAp particles be small (e.g., about 2 to 3µm) because the HAp needs to be absorbed in the body.

When a large amount of HAp is contained in the resin fiber, the fiber tends to be brittle. If the fibers become brittle, there is a risk that a roll cake formed by winding a nonwoven fabric sheet or a nonwoven fabric sheet cannot maintain a three-dimensional skeleton after implantation into a body as a scaffold material. In order to compensate for the drawback, it is proposed to reduce the content of HAp or increase the molecular weight of the resin used for the fibers of the scaffold sheet.

### <Culture>

A container (or well) is filled with a medium for proliferating adipose-derived stem cells (ADSC medium). Then, a nonwoven fabric sheet is placed from above a piece of adipose tissue containing adipose-derived stem cells, and completely immersed in a ADSC medium filled in the container.

Mechanical stress is applied to the adipose tissue by, for example, placing a weight on it so that the adipose tissue and the nonwoven fabric sheet are brought into close contact with each other by receiving pressure from above the nonwoven fabric sheet. By placing a nonwoven fabric sheet in a condition in which adipose tissue is seeded and trapped with an eye dish or the like and pressing it, it becomes possible to increase the degree of adhesion between adipose tissue and fibers and promote the stem cells contained in adipose tissue to crawl out onto the surface of the fibers.

In this case, the nonwoven fabric sheet may be placed only from above the adipocyte tissue mass, or adipose tissue mass may be sandwiched from both upper and lower directions. When a nonwoven sheet is placed only on the upper side of the adipose tissue mass, the lower side of the adipose tissue mass contacts the bottom surface of the cell strainer or culture vessel, where the stem cells may crawl out of the adipose tissue. By sandwiching the adipocyte tissue from both upper and lower sides by nonwoven fabric sheets, it becomes possible to prevent the stem cells contained in the adipocyte tissue from escaping into the cell strainer or the bottom surface of the culture container and let the stem cells crawl out into the nonwoven fabric sheet scaffold.

As another preferred embodiment, as shown in FIG. 13, a laterally elongated nonwoven fabric sheet is wound to form a roll cake and the roll cake is completely immersed in a container filled with medium for adipose derived stem cell proliferation. Using non-woven sheets formed in a roll cake, pressure applied to tighten the non-woven sheets and cell tissues can be controlled by pulling the ends of the roll cake to adjust the force of sandwiching the adipose derived stem cells. By this method, proliferation of the cells can be controlled.

By applying a pressure from outside of the nonwoven sheet, cell growth of the adipose-derived stem cells existing in the adipose tissue is dramatically accelerated, resulting in large amounts of adipose tissue-derived stem cells is obtained by initial cultures, and thus passage culture becomes not necessary.

When the scaffold used in the present invention is configured in a roll cake shape as shown in FIG. 13, the adipose-derived stem cells can be produced into a three-dimensional configuration having a desired shape and dimension by adjusting the size, fiber diameter, inter-fiber distance, and the like of the nonwoven fabric sheet for producing the roll cake. In the sandwich or roll cake system by two nonwoven fabric sheets, adipose tissue is firmly sandwiched in a gap between the nonwoven fabric sheets, so that adipose tissue is allowed to migrate (crawl out) in both sides direction, and extraction efficiency can be increased. Cell growth can be controlled by adjusting the force of sandwiching the adipose derived stem cells. A nonwoven fabric sheet or a roll cake prepared by winding a nonwoven fabric sheet can be used as a seedling bed of stem cells. It can be bent, folded, crushed, or cut to suit a defect portion depending on the necessity. It is also possible to tether a plurality of nonwoven fabric sheet or roll cakes. In this way, it becomes possible to freely make large tissues that cannot be reached by conventional cell sheet.

### <graft>

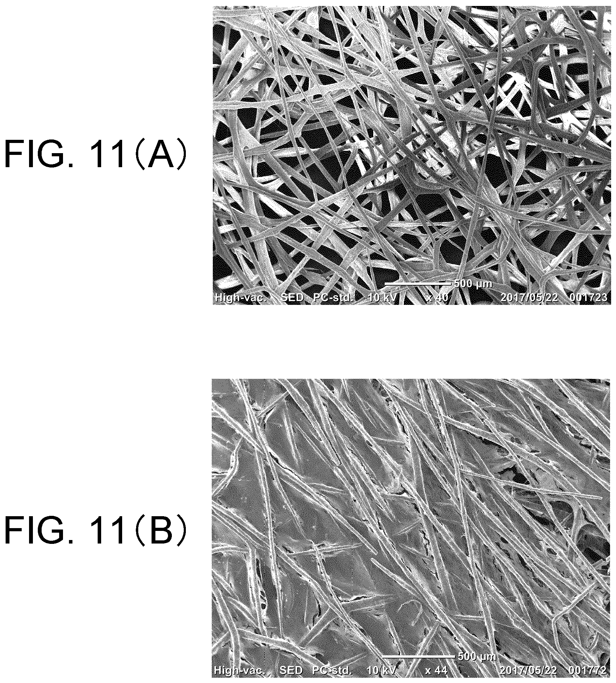

By using the nonwoven fabric sheet formed of biodegradable fibers of present invention as a scaffold, adipose-derived stem cells are proliferated on the scaffold in vitro such that adipose stem cells are grown between the fibers to achieve the state in which the space between the fibers are filled by the adipose stem cells (confluent state) (see FIG. 11). Upon reaching this state, the proliferating adipose derived stem cells can be implanted into the patient's body together with the scaffold of nonwoven sheet. In this case, because use of trypsin is not needed, the cells can be implanted into a human body without damaging the cells.

As another grafting method, after proliferating adipose-derived stem cells using the scaffold material made of a nonwoven fabric sheet, adipose-derived stem cells are detached from the scaffold using trypsin having an adjusted concentration, and then the detached adipose derived stem cells can be grafted into the body.

The nonwoven sheets used as a scaffold for cell proliferation contains adipocyte masses collected from a patient, along with proliferated adipose tissue-derived stem cells. The adipose tissue masses can be implanted into a body of a patient together with the nonwoven sheet that contains proliferated adipose stem cells. Alternatively, pinched adipose tissue mass is removed from the scaffold using a tweezer so that only the nonwoven fabric sheet and the proliferated adipose stem cells can be grafted.

### [Embodiment]

Hereinafter, a method of extracting and culturing adipose derived stem cells using the kit for stem cell extraction culture of the present invention will be described with reference to the accompanying drawings.

### <Configuration of the Kit for Extracting and Culturing Adipose Tissue-Derived Stem Cells of the Present Invention>

- Stem cell extraction culture sheet
- Human adipose tissue-derived stem cell culture medium (preferably with serum)
- Cell strainer (e.g. Model No. 22-363-549, Thermo Fisher Scientific Co., Ltd.)
- Glass eye dishes or glass rings (recommended for use in degassing and culture without cell strainer)
   e.g. glass eye dish 20 (thickness: 3 mm, hole diameter: 2 mm) Toshin Riko Co., Ltd.
   e.g. cloning ring AGC Techno Glass, Inc. Model Number RING-12
- Culture vessel (6-well plate, 10 cm dish, 15 cm dish, etc.)
   e.g. 6-well multi-well plate coning model number 3516
   e.g. Cell culture dish 100 mm BM Device Model No. 93100
   e.g. adhesive cell culture plate dish 150 Sumitomo Bakelite Corporation Model No. MS-10150

### <Selection of culture method>

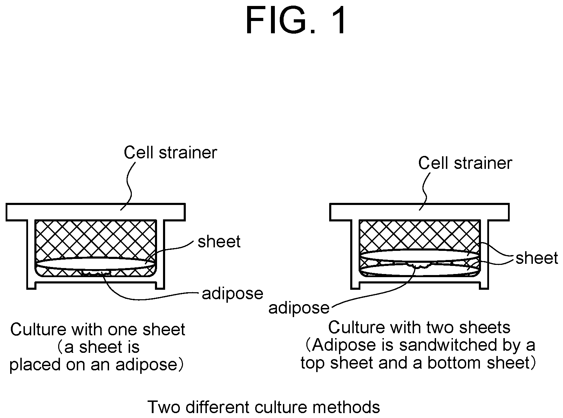

Culture is carried out by placing a sheet seeded with adipose tissue in a cell strainer. A method of culturing using a single sheet (placing a sheet on top of adipose tissue mass) and a method of culturing using two sheets (sandwiching adipose tissue mass between two sheets from top and bottom) are both possible (see Fig. 1). It is also possible to use more number of sheets by laminating multiple sheets.

### <Select culture vessel>

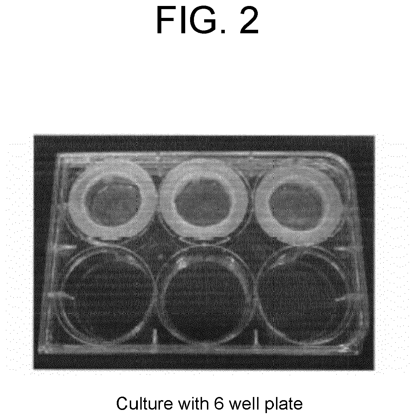

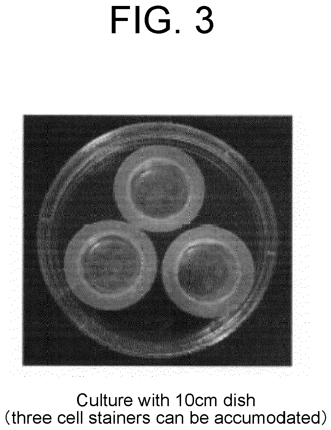

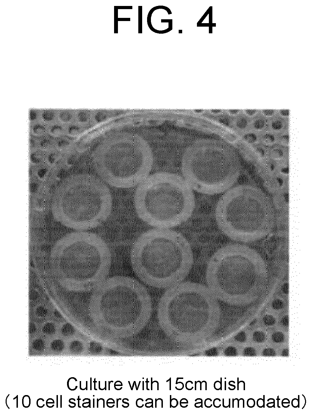

Select culture vessels from 6-well plates, 10 cm dishes, and 15 cm dishes. (See Figs. 2, 3 and 4). The maximum number of samples per culture vessel and the recommended volume of medium are shown in Table 1 below.

**Table 1 Maximum number of samples for each culture container and amount of culture medium (recommended)**

| Culture container | Maximum number of samples | Amount of culture medium |
|---|---|---|
| 6 well plate | 6 | 8 ml for each sample |
| 10 cm dish | 3 | 25 ml |
| 15cm dish | 10 | 60 ml |

### <Sheet Degassing>

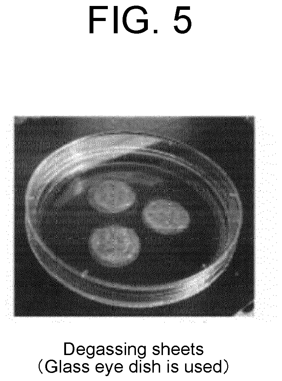

When the sheet is to be submerged in the medium, bubbles are generated in the voids of the nonwoven fabric sheet, and thus, there is a risk that the sheet floats as on the medium. In order to avoid floating the sheet on the medium, it is desirable to degas the sheet by the following procedure.
1) 8-10mL of culture medium is added into a 10 cm dish.
2) Take out required number of sheets for stem cell extract culture using a sterile tweezer and place them in a dish containing the culture medium.
3) Place a weight of sterile glass-eye dishes or glass rings from the top of the sheet so that the sheet is completely submerged in the medium. (see Figure 5)
4) Deaeration for 1 min at-0.09 MPa. If there is no barometer available, deaerate using a water flow aspirator for about 10 min.

### <Seeding adipose on a sheet and culture>

### A. Culturing using a cell strainer (using a 6-well plate)

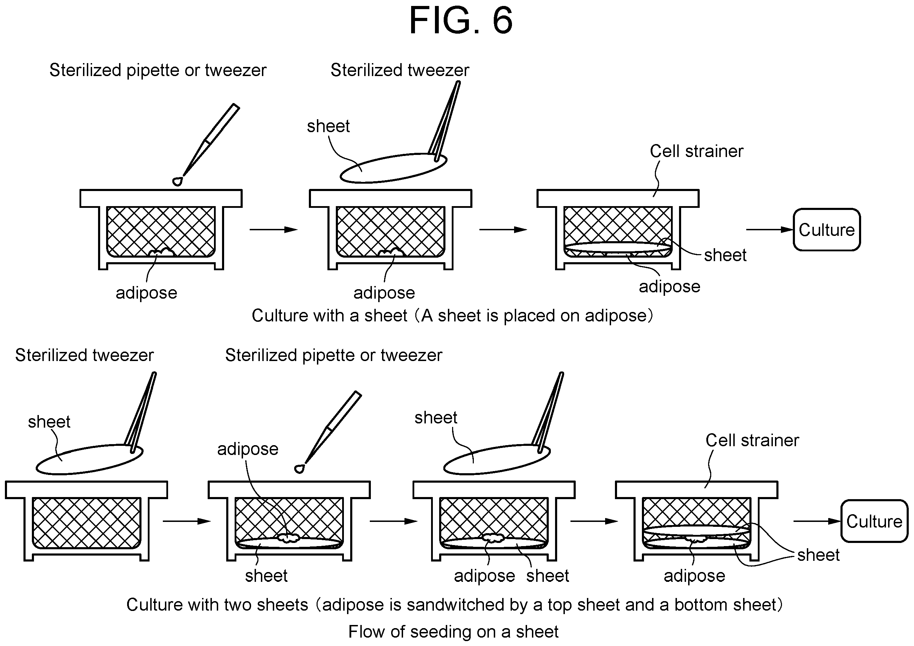

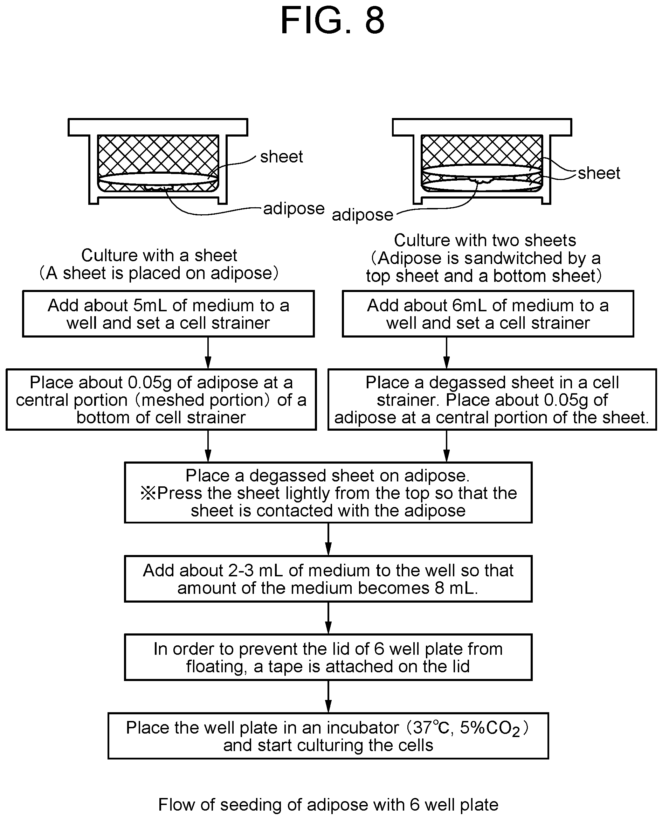

Adipose tissue masses are seeded in the procedure shown in Figures 6-8. After seeding, incubate it in an incubator (37°C, 5%CO2) to start the culturing. Replace the medium in whole or in a half every 2 to 4 days.

### Culturing with one sheet

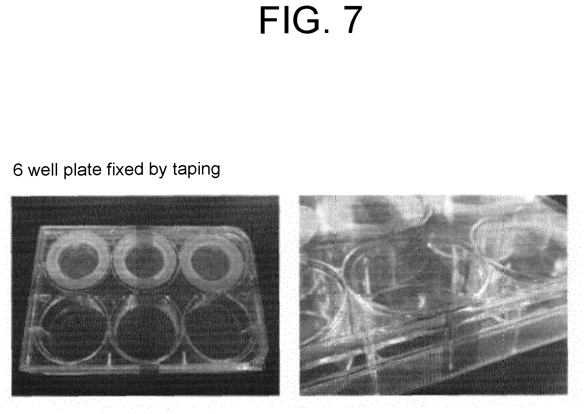

1) Add approximately 5 mL of culture medium to a well and set the cell strainer.
2) Place about 0.05 g of adipose in the middle portion (mesh portion) of the bottom of the cell strainer. In doing so, it is important to flatten the surface by pushing the bottom of the cell strainer with tweezers or the like to facilitate seeding. If the adipose floats and disperses when the adipose is placed on the bottom of the cell strainer, it is desirable to reduce the volume of the medium so that the liquid level is lowered and dispersion of adipose is prevented.
3) Place a degassed sheet from the top of the adipose.
4) Add 3 mL of culture medium to the wells so that the volume of medium in the wells is 8 mL
5) Attached a tape from the top of the lid to prevent the lid of the 6-well plate from floating. (see Figure 7)
6) Place the sheet in an incubator (37° C, 5%CO2) and start the culturing.

### Culturing with two sheets

1) Add approximately 6 mL of culture medium to the wells and set the cell strainer.
2) Place a degassed sheet in the cell strainer.
3) Place about 0.05 g of adipose tissue mass in the center of the sheet.
4) Place a degassed sheet from the top of the adipose mass.
5) Add 2 mL of medium to the wells so that the volume of medium in the wells is 8 mL.
6) Attach a tape from the top of the lid to prevent the lid of the 6-well plate from floating.
7) Place the sheets in an incubator (37° C, 5%CO2) and start incubation.

### B. Culturing without using a cell strainer (using a 6-well plate)

It is possible to culture with a sheet that is seeded with adipose without using a cell strainer. If the cell strainer is not used, there is a risk that the sheet will be misaligned. Therefore, it is desirable to press with a glass eye dish or a glass ring or the like. In this case, it should also be considered that the proportion of adipose stem cells adhering to and growing on the bottom of the well plate increases.

### Culturing with one sheet

1) Add approximately 1 mL of culture medium to the well.
2) Place one degassed sheet.
3) Place about 0.05 g of adipose in the center of the sheet.
4) Turn over the sheet so that the adipose and the well bottom are grounded.
5) Place a weight of sterile glass-grained dishes or glass rings from the top of the sheet.
6) Add 2-2.5 mL of culture medium to the wells so that the volume of medium in the well is 3-3.5 mL.
7) Place the sheet in an incubator (37° C, 5%CO₂) and start culturing.

### Culturing with two sheets

1) Add approximately 1 mL of culture medium to the well.
2) Place one degassed sheet.
3) Place about 0.05 g of adipose mass in the center of the sheet.
4) Place a degassed sheet from the top of the adipose mass.
5) Place a weight of sterile glass-grained dishes or glass rings from the top of the sheet.
6) Add 2-2.5 mL of culture medium to the well so that the volume of medium in the wells is 3-3.5 mL.
7) Place the sheet in an incubator (37° C, 5%CO₂) and start culturing.

FIG 11 illustrates the results of proliferating stem cells in a nonwoven fabric sheet in accordance with the method of the present invention using a kit of the present invention. FIG 11 (a) shows the state of the nonwoven sheet before culture, and FIG 11 (b) shows the state after culture. From FIGS. 11 (a) and (b), it can be seen that by culturing using the method of the present invention, adipose derived stem cells are proliferated in much larger amount than that of prior art to fill the space between the fibers to make a confluent state.

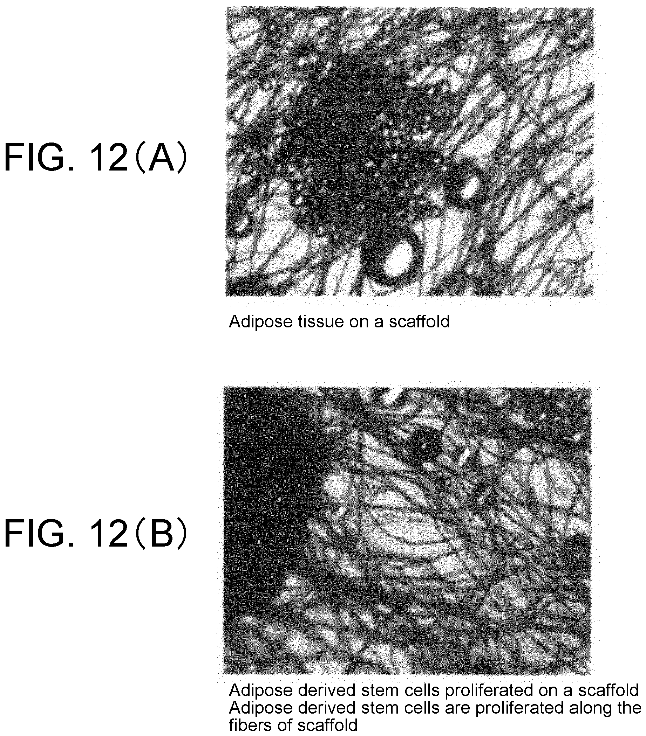

Fig. 12(a)(b) shows the results of culture of adipose cells using the adipose stem cell separation kit of Bio Mirai Kobo Co., Ltd. From the comparison of FIGS. 11 and 12, it can be seen that cell culture using the method/kit of the present invention can achieve a much larger amount of cell proliferation compared with that of the prior art.

### [Experiment]

Human adipose tissue was seeded onto the nonwoven fabric sheet of the present embodiment, and cell adhesion to the nonwoven fabric sheet was evaluated by WST-1 (absorbance) measurement.

### Condition of experiment

Nonwoven fabric sheet: a composite fiber (outer diameter: 10 to 60 µm) having a composition of PLGA50 wt%/HAp50 wt% produced by electrospinning method was collected as a nonwoven fabric sheet, and cut into a circular shape having a diameter of 23mm to obtain a sample 1
Seeded adipose: human adipose tissue (aspirated from the superficial thigh)
Culture media: ADSCADSC-GM
Culture vessel: 6-well plate
Culture medium volume (per well):10 mL
Culture temperature/ CO₂ density 37 ° C, 5%CO₂
Culture days: 4 days, 12 days, 22 days, 32 days, 42 days
Mount of seeded adipose: 0.05 g
Absorbance (wavelength 440 nm) was measured 5 times for all WST-1 measurements.

### [Result of experiment]

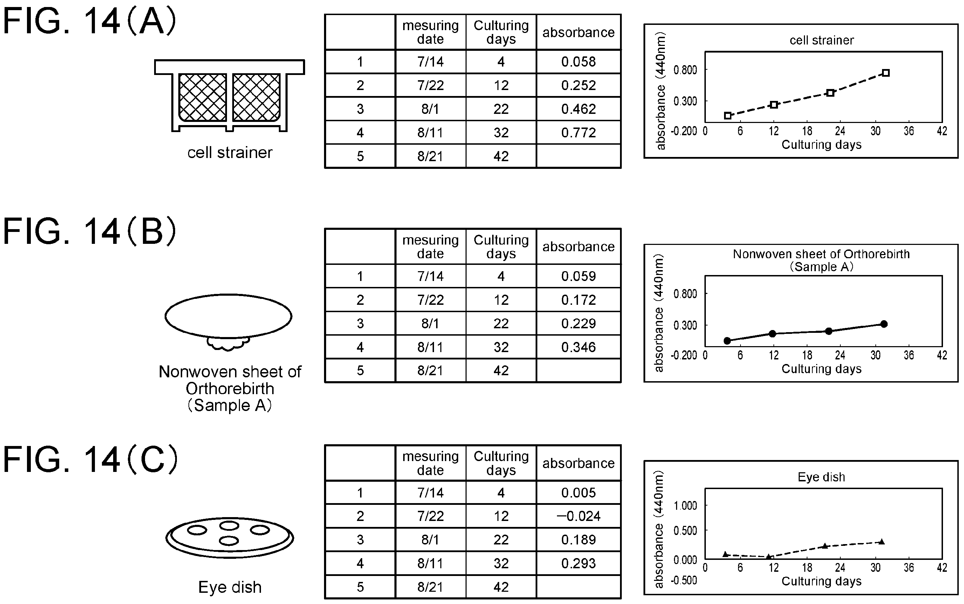

Culture days: 4 days, 12 days, 22 days, 32 days, and 42 days. The results of measurement on the cell strainer, the nonwoven fabric sheet sample 1, and the eye dish are shown in FIGS. 14 (a), 14(b), and 14(c), respectively.

### [Observation of the result of experiment]

1) As shown in FIG. 14(a), when a mass of adipose tissue was placed on the bottom of the cell strainer and a single nonwoven fabric sheet was placed to cover thereon, the stem cells crawled out on the surface of the cell strainer to which the adipose tissue was in contact. Result of adherent culture was: absorbance of 0.772 in 32 days of culture.
2) As shown in FIG. 14 (b), the result of adherent culture of stem cells crawling out from the adipose tissue mass into the nonwoven fabric sheet was: absorbance of 0.346 in 32 days of culture.
3) As shown in FIG. 14(c), the result of the stem cells that had crawled out from the addipose tissue mass into the nonwoven fabric sheet onto the eye dish placed on the nonwoven fabric sheet via the nonwoven fabric sheet and adhered and cultured there was: absorbance of 0.293 in 32 days of culture.
4) By laying a nonwoven fabric sheet on a cell strainer and placing adipose tissue mass on it and covering it with another nonwoven fabric sheet from above, it is considered that, when the nonwoven fabric sheet is immersed in a medium in a condition of being sandwiched between the nonwoven fabric sheet from above and below the adipose tissue mass, the stem cells adhering to the cell strainer decreases similarly to the culture adhered to the eye dish, and accordingly, the number of stem cells adhered and cultured on the nonwoven fabric sheet increases.
5) From Figures 14(a)-(c), it is believed that a significant amount of stem cells can be prevented from escaping into the cell strainer and adhered to the nonwoven fabric sheet by culturing the adipose tissue in a condition in which the adipose tissue is sandwiched between two nonwoven fabric sheets from above and below and sandwiched between them and then immersed in the medium.

By observing and evaluating the differentiation of adipose tissue-derived stem cells cultured using the nonwoven sheet of Sample 1, whether adipose tissue-derived stem cells proliferated in a state that maintained their differentiation potential was observed.

### Experiment of differentiation into chondrocytes

On the day prior to adipose tissue seeding, two nonwoven fabric sheets and one glass eye dish were placed on a cell strainer. and three sets of them were placed on a 6 well plate. Degassing and immersion were performed with PBS 1% PS. Next day, after receiving the adipose tissue, 0.02 g of adipose tissue was placed at the center so that the adipose tissue is sandwiched by the nonwoven fabric sheets, and culturing was performed in ADSCGM medium for 24 days while replacing the medium every three days. When the nonwoven fabric sheet became densely occupied by adipose-derived stem cells (ADSCs), the medium was replaced by a medium for differentiation into chondrocytes and conducted culturing for three weeks while replacing the medium every three days.

### Experiment of differentiation into vascular endothelial cells

On the day prior to adipose tissue seeding, two nonwoven fabric sheets and one eye glass dish were placed on a cell strainer, and three sets of them were placed on a 6 well plate, and degassing and immersion were performed with PBS 1% PS. Next day, after receiving the adipose tissue, 0.02 g of adipose tissue was placed at the center of the sheet so that the adipose tissue is sandwiched by the nonwoven fabric sheets, and culturing was performed in ADSCGM medium. After 3 days, the medium was replaced by EBM-2 medium (LONZA), and culturing was performed for 32 days while replacing the medium every 3 days.

### Experiment of differentiation into chondrocytes

On the day prior to adipose tissue seeding, two nonwoven fabric sheets and one glass eye dish were placed on a cell strainer, and three sets of them were placed on a 6 well plate, and degassing and immersion were performed with PBS 1% PS. Next day, after receiving the adipose tissue, 0.02 g of adipose tissue was placed at the center of the sheet so that the adipose tissue is sandwiched by the nonwoven fabric sheets, and culturing was performed in ADSCGM medium for forty days while replacing the medium every three days. When the nonwoven fabric sheet became densely occupied by adipose-derived stem cells (ADSCs), the medium was replaced by a medium for differentiation into adipose derived stem cells (ADSCs) and conducted culturing for one week while replacing the medium every three days.

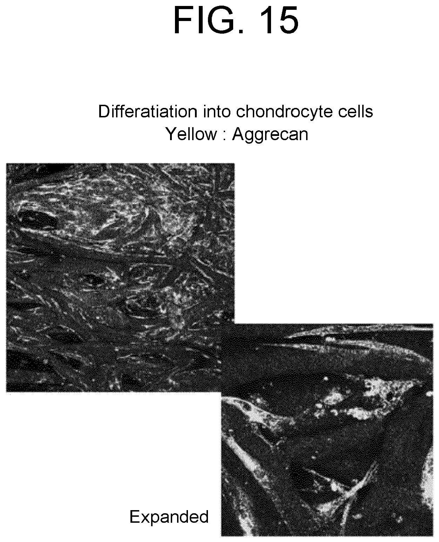

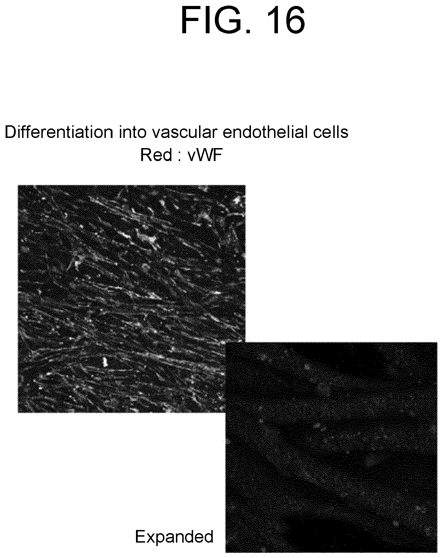

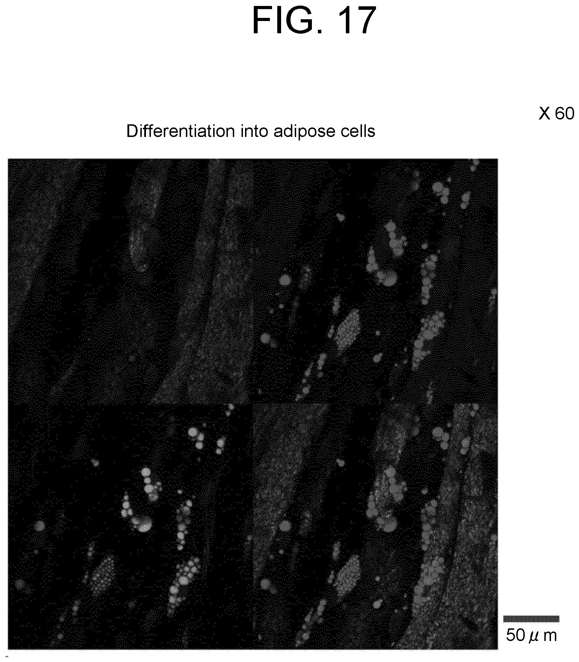

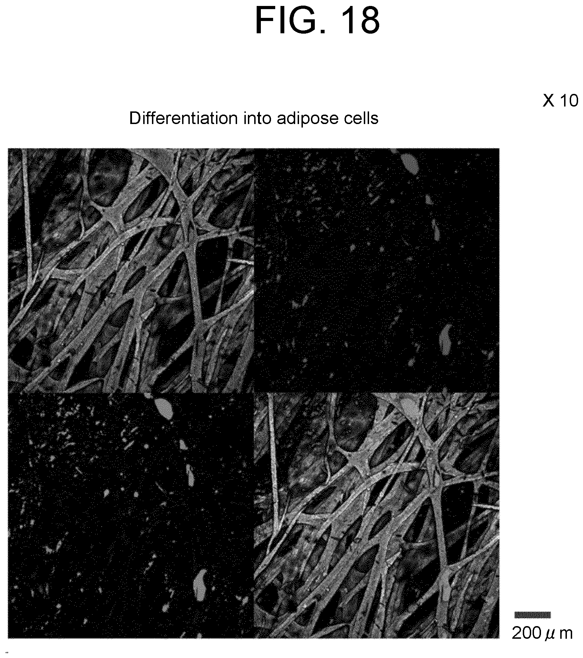

SEM photographs of the cells obtained by differentiation induction into chondrocytes are shown in FIG. 15, SEM photographs of the cells obtained by differentiation induction into vascular endothelial cells are shown in FIG. 16, and SEM photographs of the cells obtained by differentiation induction into adipocytes are shown in FIGS. 17 and 18. The results of these differentiation experiments showed that adipose derived stem cells cultured using the methods of the present invention proliferated in an undifferentiated state and possessed the ability to differentiate into chondrocytes, vascular endothelial cells, and adipocytes.

While the present invention has been described based on an embodiment in which stem cells are grown in a nonwoven fabric sheet made of biodegradable fibers and implanted in a human body together with the nonwoven sheet scaffold, the present invention is not necessarily limited to that case, and it is also possible to detach the stem cells grown in a large amount using the method and kit of the present invention from the nonwoven fabric sheet scaffold using trypsin and implant only the stem cells into a human body.
The methods and kits of the present invention can also be used to isolate and extract each somatic stem cell from not only adipose tissue but also umbilical cord tissue, skin tissue, synovial tissue, pulp tissue, bone marrow tissue, and the like. Furthermore, the nonwoven sheet used in the present invention can be used not only to extract adipose stem cells from adipose tissue, but also as a scaffold for directly seeding and culturing adipose stem cells, other somatic stem cells, iPS cells, ES cells, and the like.

## Claims

1. A method of extracting and proliferating adipose-derived stem cells in an adipose tissue without using a collagenase, the method comprising:
placing an adipose tissue mass containing adipose-derived stem cells collected from a living body of a patient in a culture vessel, and covering the adipose tissue mass by a nonwoven fabric sheet made of biodegradable fibers having an outer diameter of 10 µm to 100 µm and having a gap of 10 µm to 500 µm between the fibers;
filling the culture vessel with a culture medium for adipose-derived stem cells, and immersing the adipose tissue mass covered by the nonwoven sheet in the culture medium for adipose-derived stem cells,
pressing the nonwoven fabric sheet against the adipose tissue mass so that the adhesion between said fat tissue mass and said nonwoven fabric sheet is increased, thereby promoting the adipose-derived stem cells contained in the adipose tissue mass to crawl out to the surface of the biodegradable fibers so that the adipose-derived stem cells crawl out and proliferate in a large amount in a gap space between the fibers of the nonwoven fabric sheet.

2. The method of claim 1, wherein the biodegradable fibers are electrospun using an electrospinning method and contain 40 to 80% by weight of HAp.

3. The method according to claim 1 or 2, wherein an adhesion between the nonwoven fabric sheet and the adipocyte mass is enhanced by placing a weight from above on a nonwoven fabric sheet covering the adipose tissue mass.

4. The method according to any one of claims 1 to 3, wherein the adipose tissue mass is sandwiched between two nonwoven fabric sheets and is immersed in a medium in a sandwich state to culture the cells.

5. The method of any one of claims 1-4, wherein the adipose tissue mass is placed in a cell strainer having a mesh structure, the cell strainer containing the adipose tissue mass is placed in a culture vessel, and the culture vessel is immersed in adipose-derived stem cell culture medium to immerse the adipose tissue mass in adipose-derived stem cell culture medium.

6. The method according to any one of claims 1 to 5, wherein the adipose tissue mass is placed in a cell non-adherent plate dish, and the plate dish is filled with the culture medium to culture the cells.

7. A kit for extracting adipose-derived stem cells from adipose tissue and proliferating the adipose-derived stem cells without using collagenase, the kit comprising:
a cell culture vessel, a cell strainer, a nonwoven fabric sheet, eye plate or a ring used to press the nonwoven fabric sheet,
side and bottom faces of the cell strainer are meshed so that the cell culture medium can penetrate into the cell strainer through pores of the mesh,
the nonwoven fabric sheet is made of a biodegradable fiber having an outer diameter of 10 µm -100 µm, and a gap between the fiber is 10 µm - 500 µm,
an adipose tissue mass containing adipose-derived stem cells is placed in the cell strainer, and a culture medium for adipose-derived stem cells is filled in the culture vessel in a state that the adipose tissue mass placed in the cell strainer is covered by the nonwoven fabric sheet so the adipose cell tissue is immersed in the medium, and the non-woven sheet covered by the adipose tissue mass is pressed from the top with the eye dish or ring in that state, thereby adhesion between the adipose tissue mass and the non-woven sheet is increased, thereby the adipose-derived stem cells contained in the adipose tissue mass is promoted to crawl out onto the surface of the biodegradable fibers and the adipose-derived stem cells are proliferated in a space between the fibers of the non-woven sheet.

8. The kit for extracting adipose-derived stem cells according to claim 7, wherein the biodegradable fiber is electrospun using an electrospinning method and contains 40 to 80% by weight of HAp.

9. The kit for extracting adipose-derived stem cells according to claim 7 or 8, wherein the culture container is a non-cell adhesive plate dish.
